# EUROPEAN PATENT APPLICATION

(11) **EP 4 563 126 A2**
(43) Date of publication of application: **04.06.2025**
(21) Application number: 25157086.7
(22) Date of filing: 24.08.2020
(51) Int. Cl.: A61F 2/844

(54) **PROSTHETIC HEART VALVES**

(30) Priority: 29.08.2019 US 201962893621 P
(62) Divisional of application: 20767676.8
(71) Applicant: Edwards Lifesciences Corporation, Irvine, CA 92614 (US)
(72) Inventor: Nir, Noam, IRVINE, CA, 92614 (US)
(74) Representative: Eisenführ Speiser

(57) **Abstract**

A prosthetic valve assembly includes a radially expandable and compressible annular frame. The frame includes a plurality of interconnected struts, which include a plurality of inner struts and a plurality of outer struts. The inner struts overlap adjacent outer struts at a plurality of pivot joints. Radial expansion or compression of the annular frame causes the inner struts to pivot relative to the outer struts at the pivot joints. The assembly also includes a valvular structure inside the frame having a plurality of leaflets that regulate blood flow through the frame. Some of the struts include recessed portions and other struts that cross the recessed portions include segments that are seated in the recessed portions at the pivot joints. This provides a continuous or flush inner or outer surface of the frame.

## Description

### CROSS-REFERENCE TO RELATED APPLICATIONS

This application claims the benefit of U.S. Provisional Patent Application No. 62/893,621 filed August 29, 2019, which is incorporated by reference herein in its entirety.

### FIELD

The application relates to prosthetic heart valves, and to associated systems and methods.

### BACKGROUND

Current designs of a mechanical frame for a prosthetic heart valve include a plurality of crossing struts, attached to each other at junctions and forming a grid. The grid of struts forms the wall of the frame that is essentially cylindrical but may vary from a perfect cylinder per any specific frame design. This grid has two layers of struts -- radially inner layer and radially outer layer. All struts in each layer are essentially parallel to each other and cross the struts of the other layer. All inner faces of the inner layer struts form the inner diameter face of the frame. All outer faces of the outer layer struts form the outer diameter face of the frame.

### SUMMARY

Described herein are examples of prosthetic valves, components thereof, and related methods of assembling, delivering, and using the same. Prosthetic valves disclosed herein can be implanted within any of the native valves of the heart (e.g., the aortic, mitral, tricuspid and pulmonary valves). In some embodiments, the prosthetic valve can be delivered through the vasculature and implanted to the heart of a patient by using a delivery apparatus, such as a catheter-based delivery apparatus. Frames disclosed herein may also be used for prosthetic valves in other conduits of the body, or as stents without a valve structure.

Certain embodiments of the disclosure concern a prosthetic heart valve. The assembly can include a radially expandable and compressible annular frame. The frames can be expanded within an implant location by applying radially outward forces on the frame, such as by a balloon or mechanical means, or by applying circumferential separation forces on the frame, or by applying axial shortening forces on the frame.

The frame can include a plurality of interconnected struts. The plurality of interconnected struts can include a plurality of inner struts and a plurality of outer struts. The inner struts can overlap adjacent outer struts at a plurality of pivot joints, coupled together with pins or other fasteners that permit the struts to pivot relative to each other. The interconnected struts can be arrayed in a lattice or grid, which each strut extending diagonally or helically around the frame. Radial expansion or compression of the annular frame can cause the inner struts to pivot relative to the outer struts at the pivot joints.

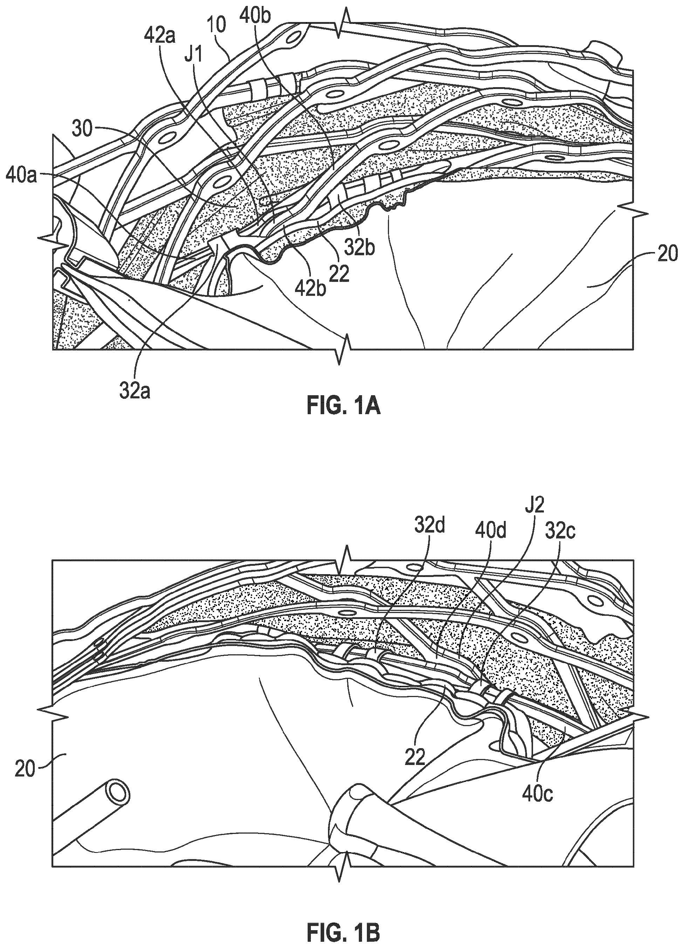

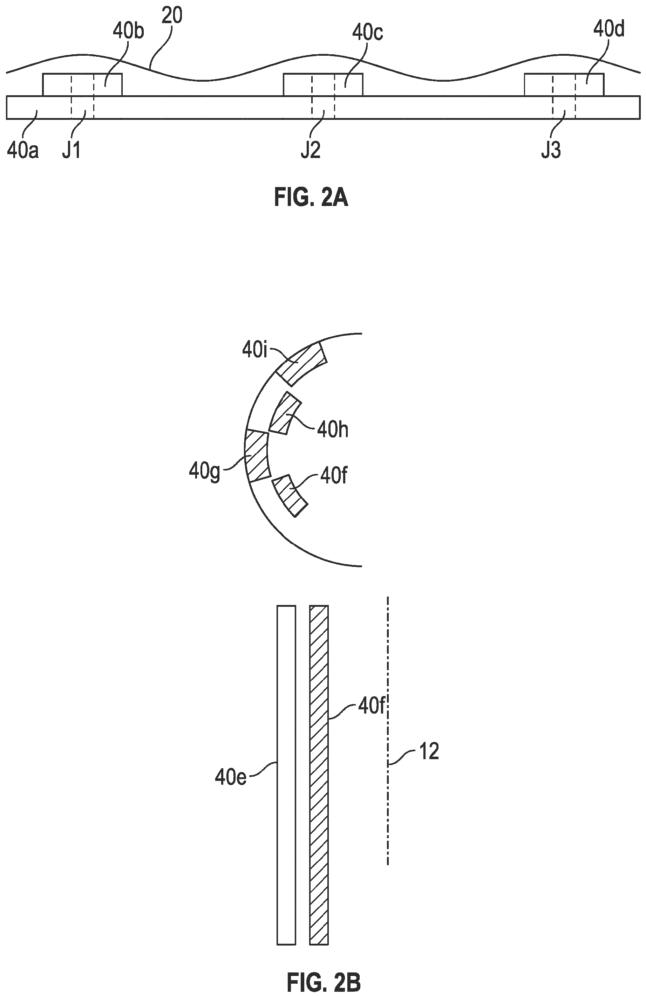

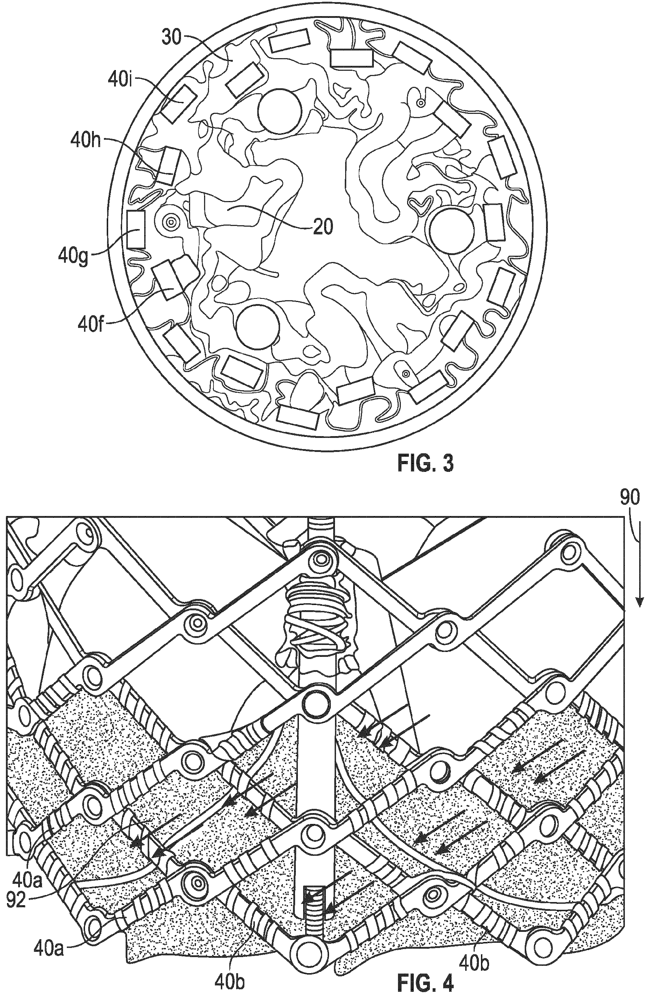

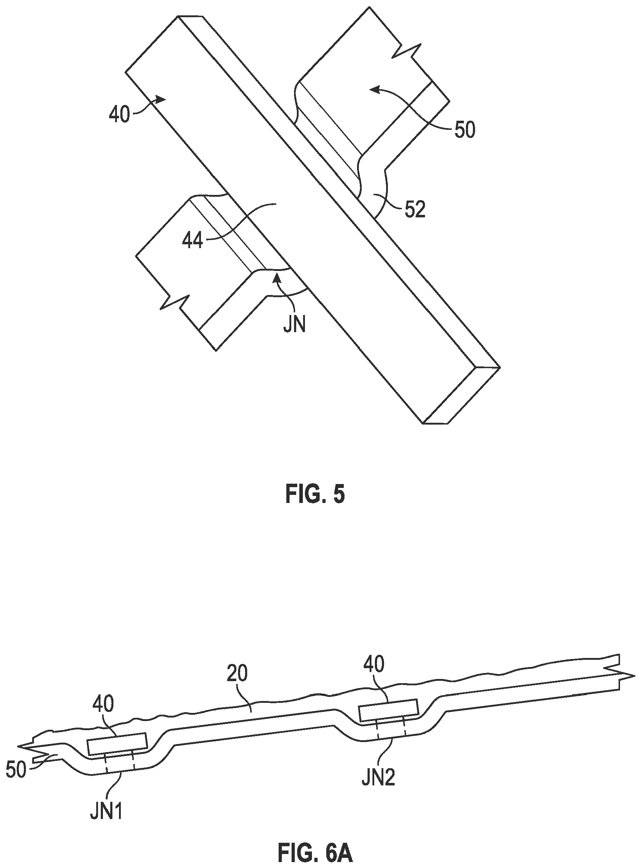

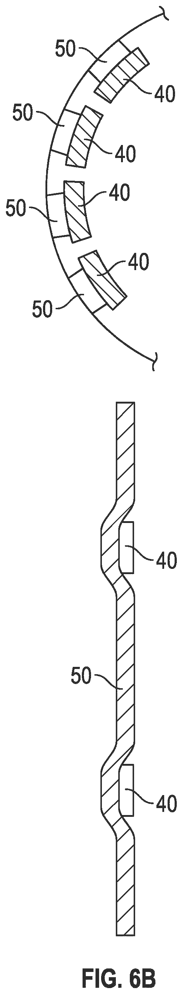

More details regarding the general structure of such prosthetic valve assemblies and mechanical frames can be found in U.S. Provisional Application 62/854,702 file on May 30, 2019, which is incorporated by reference herein in its entirety. With reference to U.S. Provisional Application 62/854,702, FIG. 1 shows an entire prosthetic valve using a mechanical frame comprising interconnected struts pivotably coupled at pivot joints, FIGS. 2A and 2B show just the frame and illustrate the radially expanded and compressed configurations, FIGS. 3 and 4 show examples of how other components are attached the frame, and FIGS. 6A and 6B show individual struts, and FIG. 6C shows a pivot joint in detail. Other components and details of the valves and frames are also described in U.S. Provisional Application 62/854,702, such as the valve leaflets, inner skirts, outer skirts, frame expansion mechanisms, delivery devices, exemplary materials and dimensions, and related methods of assembly and use. The valves and frames disclosed herein can have an analogous structure to those illustrated in U.S. Provisional Application 62/854,702, except as described herein. Further details regarding transcatheter prosthetic heart valves, including the manner in which the valvular structure can be mounted to the frame of the prosthetic valve can be found, for example, in U.S. Patent Nos. 6,730,118, 7,393,360, 7,510,575, 7,993,394, and 8,252,202, and U.S. Publication No. 2018/0325665, all of which are incorporated herein by reference in their entireties. Further details regarding the construction of frames and the prosthetic valves are described in U.S. Patent Publication Nos. 2018/0153689 and 2018/0344456, and U.S. Patent Application Nos. 16/105,353 and 62/748,284, all of which are incorporated herein by reference.

In embodiments disclosed herein, at least some of the struts comprise recessed portions at the pivot joints and segments of others of the struts are seated in the recessed portions at the pivot joints. In some embodiments, at least some of the outer struts comprise recessed portions and segments of at least some of the inner struts are seated in the recessed portions, and in some embodiments at least some of the inner struts comprise recessed portions and segments of at least some of the outer struts are seated in the recessed portions. In some embodiments, at least some of the inner struts comprise recessed portions that receive segments of the outer struts and at least some of the outer struts comprise recessed portions that receive segments of the inner struts.

The pivot joints where the segments are seated in the recessed portions can be configured such that they have a generally continuous inner or outer surface, which can facilitate attachment of leaflets or skirts to the frame and provide other benefits. In some embodiments, the pivot joints where the segments are seated in the recessed portions have a generally continuous inner surface, such that inner surfaces of the seated segments and adjacent inner surfaces the struts joined to the seated segments have about equal radial dimensions from a centerline of the frame. This can help with attachment of the leaflets and inner skirt to the inside of the frame. In some embodiments, the pivot joints where the segments are seated in the recessed portions have a generally continuous outer surface, such that outer surfaces of the seated segments and adjacent outer surfaces the struts joined to the seated segments have about equal radial dimensions from a centerline of the frame. This can help with attachment of an out skirt and/or help prevent perivalvular leakage.

The struts can comprise multiple recessed portions located at respective pivot joints and segments of all the crossing struts can be seated in the recessed portions at the respective pivot joints.

The recesses defined by the recessed portions can be sized to receive the crossing strut segments while allowing for pivoting motion between the struts. The recesses of the recessed portions can have a radial depth that is about equal to the radial thickness of the segments seated in the recessed portions, such that their radially facing surfaces can be flush or coplanar, providing a continuous overall frame surface. The recesses of the recessed portions can have a length that is greater that a width of the segments seated in the recessed portions to allow room for the seated segments to pivot within the recesses.

Also disclosed herein are methods of forming a mechanical frame by coupling inner struts to outer struts at pivot joints, such as via pivot pins. Also disclosed herein are methods of crimping or radially compressing a prosthetic heart valve including a mechanical frame wherein the struts move closer to parallel with the axial dimension in the same circumferential plane such the scissor-like shearing of the valve structure within can be avoided. Also disclosed herein are methods of implanting a prosthetic heart valve wherein the mechanical frame is radially expanded and presses evenly against surrounding native tissue to prevent paravalvular leakage paths between the frame and the native tissue.

More details of the disclosed technology are described below and with reference to the attached drawings.

### BRIEF DESCRIPTION OF THE DRAWINGS

FIGS. 1A and 1B are views from inside of a prosthetic heart valve, showing how the leaflets and skirts connect to the struts of the frame.
FIG. 2A. is a schematic views of intersections between struts of the frame, showing an uneven frame surface interfacing with a leaflet.
FIG. 2B is a schematic view of the frame illustrating how the struts can create scissor-like shearing forces.
FIG. 3 is a cross-sectional view of a heart valve illustrating radial and circumferential positioning of the inner and outer struts of the frame when the frame is crimped.
FIG. 4 shows an external side of the heart valve without an outer skirt, illustrating perivalvular leak pathways.
FIG. 5 illustrates a junction between two frame structs where one strut has a recessed portion that receives the crossing strut, providing a relatively continuous or flush overall surface.
FIG. 6A shows a pair of strut junctions like in FIG. 5, where the continuous surface is an inner surface facing a valve leaflet.
FIG. 6B is a schematic view of a frame incorporating recessed strut junctions like in FIG. 5, illustrating alignment between the struts that mitigates shearing forces on the leaflets and skirts.

### DETAILED DESCRIPTION

Described herein are examples of prosthetic heart valves that can be implanted within any of the native valves of the heart (e.g., the aortic, mitral, tricuspid and pulmonary valves). The present disclosure also provides frames for use with such prosthetic implants. Prosthetic valves disclosed herein can be radially compressible and expandable between a radially compressed state and a radially expanded state. Thus, the prosthetic valves can be retained by an implant delivery apparatus in the radially compressed state during delivery, and then expanded to the radially expanded state once the prosthetic valve reaches the implantation site.

Conventional designs of a mechanical frame for a prosthetic heart valve present several disadvantages, which can include some or all of the following, among others: (1) Each leaflet can be connected to an inner strut at one edge, and to an outer strut at its other edge. The edge of the leaflet connected to the inner strut can be attached to a uniform surface, while the edge connected to an outer strut is on a non-uniform surface (e.g., "zig-zags" along a changing diameter relative to the center of the frame, since it must "climb" over the crossing inner struts). Such a pattern can affect the accuracy and uniformity of leaflet attachment because: (a) it can create folds in the leaflet that alter its shape and interfere with its motion ability, and (b) attachment to different diameters can create asymmetric leaflets such that leaflets coaptation is less than optimal. (2) The total wall thickness of the frame can be equal to or greater than twice the thickness of a strut, which is a significant factor in crimping. (3) During the process of crimping, the relative movement between adjacent inner and outer struts exerts scissor-like shearing forces that can compromise the integrity of the leaflet tissue. (4) In designs that do not include an outer cloth component or a perivalvular seal component, it is likely to result in the parallel outer struts forming channels of perivalvular leakage

This application discloses a new strut geometry that can avoid the above disadvantages. Valve frames disclosed herein can include struts that have recessed intermediate segments that are configured to accommodate a respective straight intermediate segment of an adjacent crossing strut. Junctions between the crossing struts may bulge along only one surface (outer or inner) of the frame, while the opposite surface remains relatively flat or continuous.

Determination of which surface is provided as a flat/continuous surface, either the inner surface of the frame (interfacing with the leaflets) or the outer surface (interfacing with the anatomic surrounding), can be based on the arrangement of the crossing struts. In some embodiments, the outer struts can be formed with recessed intermediate segments, overlaid over "straight" inner struts, so as to form an inner continuous surface, thereby enabling both edges of the leaflets to attach to continuous inner surfaces of an equal diameter. In other embodiments, the inner struts can be formed with recessed intermediate segments, overlaid over "straight" outer struts, so as to form an outer continuous surface, thereby enabling providing additional free luminal volume in the crimped frame, to accommodate inner soft components.

Recessed portions of a strut can be formed by punching a straight strut at the desired regions. Both geometries (of a continuous outer surface or a continuous inner surface) can provide a tight interface with the anatomy of the blood vessel's wall, resulting in a one layer grid that does not form leak channels.

Figs. 1A and 1B show a frame 10 of a prosthetic heart valve comprising a plurality of interconnected struts 40a, 40b, 40c, 40d, etc. Figs. 2A and 2B show schematic representations of the frame 10. The struts each include a plurality of segments disposed between two end portions thereof, and intermediate segments 42a, 42b, etc. disposed between each couple of adjacent segments in a strut. The end portions and the intermediate segments of each strut can comprise apertures, through which the struts can be connected to each other, for example via fasteners such as pins passing through the apertures, to form pivot junctions J1, J2, etc. As can be seen in Figs. 1A-1B, the width of the junctions J1, J2 is twice the width of a single strut, and the resulting frame geometry forms uneven outer and/or inner surfaces, wherein the junctions bulge radially relative to the straight segments of the struts. Fig. 2A illustrates how the frame bulges at junctions J1, J2, J3.

Figs. 1A-1B show a valve leaflet 20 attached to a skirt portion or a cloth at scallop suture line 22, and an outer skirt 30, wherein the leaflet 20 and the outer skirt 30 are attached to an outer strut 40a of the frame 10 via suture loops 32a, 32b, etc., and wherein the leaflet 20 contacts an inner surface of the frame 10 and the outer skirt 30 contacts an outer surface of the frame 10. Fig. 1A shows an example of an overlapping outer strut 40a and inner strut 40b, connected to each other at a junction J1, such that the junction J1 bulges radially inwards, i.e. towards a centerline 12 (see Fig. 2B) of the frame 10. An edge of the leaflet 20 is attached via suture loops 32a, 32b to the outer strut 40a, and the radial inward bulging of junction J1 between the suture loops 32a and 32b forms a noncontinuous interface between the leaflet 20 and the strut 40a.

Fig. 1B shows an example of overlapping inner strut 40c and outer strut 40d connected to each other at a pivot junction J2, such that the junction J2 bulges radially outwards. An edge of the leaflet 20 is attached via suture loops 32c, 32d to the inner strut 40c, and a continuous interface is formed between the leaflet 20 and the strut 40a at this region. However, the radially outward bulging of junction J2 between the suture loops 32c and 32d creates a non-continuous interface between the outer skirt 30 and the strut 40c.

Fig. 2B shows a further challenge of the structure of the frame 10, wherein during crimping of the frame 10, the position of adjacent struts 40, such as struts 40f, 40g, 40g, 40i, may change such that they approach each other while the frame 10 is compressed or crimped, thereby creating undesired folds of either the leaflets 20 or the outer skirt 30 attached thereto, and may even exert scissor-like shearing forces that may further damage the leaflets 20 or outer skirt 30.

Fig. 3 shows a cross-sectional view of the radial displacement of adjacent struts 40f, 40g, 40g, 40i during compression of the valve, which may exert shearing forces that may damage the tissues of the leaflets 20 or the skirt 30.

Fig. 4 shows an exemplary prosthetic heart valve wherein the outer skirt is not included. The leaflets in the embodiment of Fig. 4 are connected to the frame via an inner skirt. A disadvantage of this design is that leak channels may form between parallel outer struts 40a and the surrounding blood vessel wall the valve is expanded against, directing blood flow through such channels in the direction of arrows 92, thereby resulting in an overall perivalvular leak flow in the direction of arrow 90.

Novel frames disclosed herein can provide an improved strut geometry wherein some or all of the intermediate strut segments are bent to form recessed intermediate segments, configured to accommodate straight or also bent intermediate segments of overlapping struts.

Fig. 5 shows a schematic representation of a straight strut segment 40 overlaid over a bent strut 50 that has a recessed intermediate segment 52, such that an intermediate segment 44 of the straight strut is received within and coupled to the recessed intermediate segment 52 at a junction JN. Though not shown in Fig. 5, each end portion of the bent strut 50 can also include a recessed end portion.

According to some embodiments, recessed intermediate segments 52 or recessed end portions can be formed by providing a straight strut and punching it (e.g., plastically deforming it) at the regions of such desirable recesses. Advantageously, relatively straight struts are overlaid over struts having recessed intermediate segments such that at least one surface of the resulting frame is continuous or flush, such that the continuous surface does not include any substantial radial protrusion at the junctions JN (as well as at the apices).

As shown schematically in Fig. 6A, a strut 50 having recessed intermediate segments is coupled to struts 40, such that while the junctions JN1 and JN2 bulge towards one side (lower side in this view, radially outer side of frame), the surface of the opposite side (upper side in this view, radially inner side of frame) remains substantially continuous adjacent to the leaflet 20. According to some embodiments, a frame is formed by connecting a set or layer of parallel outer struts 50 having recessed intermediate segments 52, with a set or layer of parallel crossing inner (straight) struts 40, in the manner shown in FIG. 6A, such that the inner surface of the frame is substantially continuous adjacent to the leaflet 20. Advantageously, the leaflets 20 can be connected via both of the edges to a continuous inner surface of the frame in such a geometry.

According to some embodiments, a frame is formed by connecting a set or layer of parallel inner struts 50 having recessed intermediate segments 52, with a set or layer of parallel crossing outer (straight) struts 40, such that the outer surface of the frame is substantially continuous. Advantageously, additional free inner volume may be achieved in the crimped frame of such a geometry, to accommodate inner soft components such as the leaflets and the inner skirt.

A further advantage of the disclosed embodiments is that they can enable adoption of a prosthetic heart valve that lacks an outer skirt, without the risk of leak channels forming along the outer surface of the frame. This may be accomplished at both proposed assemblages mentioned above, such that: (a) if the outer surface is continuous (i.e. junctions JN are bulging inwardly towards the lumen of the frame), no space will be formed between a frame fully expanded against the blood vessel's wall, thereby avoiding formation of leak channels, and (b) if the inner surface is continuous (i.e. junctions JN are bulging outwards the blood vessel's wall), the frame can be further expanded such that the junctions JN press against the blood vessel's wall (i.e. slightly penetrate the inner surface of the blood vessel), until the segments of the struts are tightly pressed against the blood vessel's wall, thereby avoiding formation of leak channels there between.

A further advantage of the disclosed technology is that the risk of shear forces acting on the leaflet or the skirt (see e.g., Figs. 2B and 3) during compression or crimping is mitigated. As shown in Fig. 6B, as adjacent struts 40 and 50 are approaching each other along the same circumferential plane during frame compression, thereby avoiding shearing of the soft layers disposed there between while substantially avoiding the radial displacement between adjacent struts which may result in detrimental shear or "scissor"-like forces.

### General Considerations

It should be understood that the disclosed embodiments can be adapted for delivering and implanting prosthetic devices in any of the native annuluses of the heart (e.g., the aortic, pulmonary, mitral, and tricuspid annuluses), and can be used with any of various delivery devices for delivering the prosthetic valve using any of various delivery approaches (e.g., retrograde, antegrade, transseptal, transventricular, transatrial, etc.).

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth herein. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." As used herein, "and/or" means "and" or "or", as well as "and" and "or". Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same.

The valves and frames disclosed herein are described using an axial direction defined by the centerline of the annular frame and the overall bloodflow direction from an inflow end to an outflow end, a radial direction that is defined as radiating perpendicularly from the centerline of the frame, and a circumferential direction that is perpendicular to the axial and radial directions and extends around the centerline of the frame. The term "inner" refers to objects, surfaces and areas proximal to the centerline of the frame and the term "outer" refers objects, surfaces and areas that are farther from the centerline of the frame.

In view of the many possible embodiments to which the principles of the disclosed technology may be applied, it should be recognized that the illustrated embodiments are only preferred examples of the technology and should not be taken as limiting the scope of the technology. Rather, the scope of the disclosed technology is at least as broad as the following claims and their equivalents.

The application further comprises the following embodiments:
1. A prosthetic heart valve comprising:
   a radially expandable and compressible annular frame comprising a plurality of struts, the plurality of struts comprising inner struts and outer struts that are discrete from one another, wherein the outer struts overlap adjacent inner struts at pivot joints, and radial expansion or compression of the annular frame causes the inner struts to pivot relative to the outer struts at the pivot joints; and
   a valvular structure mounted within the frame that regulates blood flow through the prosthetic heart valve;
   wherein at least some of the plurality of struts comprise recessed portions at the pivot joints, and segments of others of the plurality of struts are seated in the recessed portions at the pivot joints.
2. The valve of embodiment 1, wherein at least some of the outer struts comprise recessed portions, and segments of at least some of the inner struts are seated in the recessed portions.
3. The valve of embodiment 1, wherein at least some of the inner struts comprise recessed portions, and segments of at least some of the outer struts are seated in the recessed portions.
4. The valve of embodiment 1, wherein at least some of the inner struts comprise recessed portions that receive segments of the outer struts, and at least some of the outer struts comprise recessed portions that receive segments of the inner struts.
5. The valve of any one of embodiments 1-4, wherein the pivot joints where the segments are seated in the recessed portions have a substantially continuous inner or outer surface.
6. The valve of embodiment 5, wherein the pivot joints where the segments are seated in the recessed portions have a generally continuous inner surface, such that inner surfaces of the seated segments and adjacent inner surfaces the struts joined to the seated segments have about equal radial dimensions from a centerline of the frame.
7. The valve of embodiment 5, wherein the pivot joints where the segments are seated in the recessed portions have a generally continuous outer surface, such that outer surfaces of the seated segments and adjacent outer surfaces the struts joined to the seated segments have about equal radial dimensions from a centerline of the frame.
8. The valve of any one of embodiments 1-7, wherein at least some of the struts comprise two or more recessed portions located at respective pivot joints and segments of others of the struts are seated in the two or more recessed portions at the respective pivot joints.
9. The valve of any one of embodiments 1-8, wherein the frame further comprises pins that couple the recessed portions to the segments seated in the recessed portions at the pivot joints.
10. The valve of any one of embodiments 1-9, wherein the recessed portions have a depth that is about equal to a thickness of the segments seated in the recessed portions.
11. The valve of any one of embodiments 1-10, wherein the recessed portions have a width that is greater that a width of the segments seated in the recessed portions.
12. The valve of any one of embodiments 1-11, wherein leaflets of the valvular structure are attached to the struts that include the recessed portions.
13. The valve of any one of embodiments 1-12, wherein at least some of the struts comprise recessed end portions and end segments of others of the struts are seated in the recessed end portions forming pivot joints at apices of the frame.
14. A mechanical frame for a prosthetic valve, comprising:
   a plurality of interconnected struts that form an annular lattice that is radially expandable and compressible, the plurality of interconnected struts comprising a set of inner struts that are generally parallel to one another and a set of outer struts that are generally parallel to one another, wherein the outer struts overlap adjacent inner struts at pivot joints, and radial expansion or compression of the annular lattice causes the inner struts to pivot relative to the outer struts at the pivot joints;
   wherein at least some of the interconnected struts comprise recessed portions at the pivot joints and segments of others of the interconnected struts are seated in the recessed portions at the pivot joints.
15. The frame of embodiment 14, wherein at least some of the outer struts comprise recessed portions and segments of at least some of the inner struts are seated in the recessed portions.
16. The frame of embodiment 14 or embodiment 15, wherein at least some of the inner struts comprise recessed portions and segments of at least some of the outer struts are seated in the recessed portions.
17. The frame of any one of embodiments 14-16, wherein at least some of the inner struts comprise recessed portions that receive segments of the outer struts, and at least some of the outer struts comprise recessed portions that receive segments of the inner struts.
18. The frame of any one of embodiments 14-17, wherein the pivot joints where the segments are seated in the recessed portions have a generally continuous inner or outer surface.
19. The frame of embodiment 18, wherein the pivot joints where the segments are seated in the recessed portions have a generally continuous inner surface, such that inner surfaces of the seated segments and adjacent inner surfaces of the struts joined to the seated segments have about equal radial dimensions from a centerline of the frame.
20. The frame of embodiment 18, wherein the pivot joints where the segments are seated in the recessed portions have a generally continuous outer surface, such that outer surfaces of the seated segments and adjacent outer surfaces the struts joined to the seated segments have about equal radial dimensions from a centerline of the frame.
21. The frame of any one of embodiments 14-20, wherein at least some of the interconnected struts comprise two or more recessed portions located at respective pivot joints and segments of others of the interconnected struts are seated in the two or more recessed portions at the respective pivot joints.
22. The frame of any one of embodiments 14-21, wherein the frame further comprises pins that couple the recessed portions to the segments seated in the recessed portions at the pivot joints.
23. The frame of any one of embodiments 14-22, wherein the recessed portions have a depth that is about equal to a thickness of the segments seated in the recessed portions.
24. The frame of any one of embodiments 14-23, wherein the recessed portions have a width that is greater that a width of the segments seated in the recessed portions.
25. The frame of any one of embodiments 14-24, wherein at least some of the struts comprise recessed end portions and end segments of others of the struts are seated in the recessed end portions forming pivot joints at apices of the frame.
26. A method of forming a mechanical frame for a prosthetic valve, the method comprising:
   coupling a set of inner struts to a set of outer struts at a plurality of pivot joints to form an annular lattice that is radially expandable and compressible, wherein the inner struts are generally parallel to one another and the outer struts are generally parallel to one another, wherein the outer struts overlap the inner struts at the pivot joints, such that radial expansion or compression of the annular lattice causes the inner struts to pivot relative to the outer struts at the pivot joints;
   wherein either:
      the outer struts comprises recessed portions at the pivot joints and straight segments of inner struts are seated in the recessed portions of the outer struts at the pivot joints; or
      the inner struts comprises recessed portions at the pivot joints and straight segments of outer struts are seated in the recessed portions of the inner struts at the pivot joints.
27. The method of embodiment 26, wherein the coupling comprises attaching the straight segments to the recessed portions with pivot pins to form the pivot joints.
28. The method of embodiment 26 or embodiment 27, wherein the outer struts comprises recessed portions at the pivot joints and straight segments of inner struts are seated in the recessed portions of the outer struts at the pivot joints.
29. The method of embodiment 28, wherein the pivot joints have a substantially continuous radially inner surface.
30. The method of embodiment 28 or embodiment 29, wherein inner surfaces of the straight segments and adjacent inner surfaces of the outer struts have about equal radial dimensions from a centerline of the frame.
31. The method of embodiment 26 or embodiment 27, wherein the inner struts comprises recessed portions at the pivot joints and straight segments of outer struts are seated in the recessed portions of the inner struts at the pivot joints.
32. The method of embodiment 31, wherein the pivot joints have a substantially continuous radially outer surface.
33. The method of embodiment 31 or embodiment 32, wherein outer surfaces of the straight segments and adjacent outer surfaces the inner struts have about equal radial dimensions from a centerline of the frame.
34. The method of any one of embodiments 26-33, wherein at least some of struts comprise two or more recessed portions located at respective pivot joints and segments of others of the struts are seated in the two or more recessed portions at the respective pivot joints.
35. The method of any one of embodiments 26-34, wherein the recessed portions have a depth that is about equal to a thickness of the straight segments seated in the recessed portions.
36. The method of any one of embodiments 26-35, wherein the recessed portions have a width that is greater that a width of the straight segments seated in the recessed portions.
37. The method of any one of embodiments 26-36, wherein at least some of the struts comprise recessed end portions and end segments of others of the struts are seated in the recessed end portions and joined by pivot pins, forming pivot joints at apices of the frame.
38. A method of radially compressing a prosthetic heart valve, comprising:
   causing a set of outer struts of a mechanical frame of the prosthetic heart valve and a set of inner struts of the mechanical frame to pivot relative to one another at a plurality of pivot joints where the outer struts overlap the inner struts, such that outer struts and the inner struts become closer to parallel with an axial dimension of the frame and a radius of the frame reduces;
   wherein the outer struts comprise recessed portions at the pivot joints, and segments of inner struts are seated in the recessed portions of the outer struts at the pivot joints, such that radially inner surfaces of the outer struts between the pivot joints have an inner radius that is equal to radially inner surfaces of the inner struts between the pivot joints;
   wherein reduction of the radius of the frame causes radial compression of a valvular structure mounted within the frame; and
   wherein the radially inner surfaces of both the outer struts and the inner struts between the pivot joints maintain a substantially continuous radial contact relative to the valvular structure as the outer and inner struts become closer to parallel with the axial dimension of the frame.
39. The method of embodiment 38, wherein the substantially continuous radial contact relative to the valvular structure prevents the pivoting struts from applying shearing forces on the valvular structure during radial compression of the prosthetic heart valve.
40. The method of embodiment 38, wherein the inner surfaces of the seated segments of the inner struts and adjacent inner surfaces the outer struts joined to the seated segments have about equal radial dimensions from an axial centerline of the frame.
41. The method of any one of embodiments 38-40 wherein the outer struts each comprise two or more recessed portions located at respective pivot joints and the inner struts are seated in the two or more recessed portions of the outer struts at respective pivot joints.
42. The method of any one of embodiments 38-41, wherein the frame further comprises pins that couple the recessed portions of the outer struts to the seated segments of the inner struts at the pivot joints.
43. The method of any one of embodiments 38-42, wherein the recessed portions have a depth that is about equal to a thickness of the segments seated in the recessed portions.
44. The method of any one of embodiments 38-43, wherein the recessed portions have a width that is greater that a width of the segments seated in the recessed portions.
45. The method of any one of embodiments 38-44, wherein leaflets of the valvular structure are attached to the outer struts between the pivot joints.
46. The method of any one of embodiments 38-45, wherein at least some of the outer struts comprise recessed end portions and end segments of the inner struts are seated in the recessed end portions forming pivot joints at apices of the frame.
47. A method of implanting a prosthetic heart valve, comprising:
   positioning the prosthetic heart valve at a native heart valve region of a heart in a radially compressed state; and
   causing a set of outer struts of a mechanical frame of the prosthetic heart valve and a set of inner struts of the mechanical frame to pivot relative to one another at a plurality of pivot joints where the outer struts overlap the inner struts, such that outer struts and the inner struts become farther from parallel with an axial dimension of the frame and the frame radially expands;
   wherein the inner struts comprise recessed portions at the pivot joints, and segments of outer struts are seated in the recessed portions of the inner struts at the pivot joints, such that radially outer surfaces of the inner struts between the pivot joints have an outer radius that is equal to radially outer surfaces of the outer struts between the pivot joints; and
   wherein radial expansion of the frame causes the radially outer surfaces of the outer struts and the inner struts to evenly press against native tissue of the native heart valve region and together form a paravalvular seal against the native tissue when the frame is radially expanded.
48. The method of embodiment 47, wherein the paravalvular seal prevents paravalvular leakage of blood between the frame and the native tissue.
49. The method of embodiment 47 or embodiment 48, wherein the radially outer surfaces of the seated segments of the outer struts and adjacent outer surfaces the inner struts joined to the seated segments have about equal radial dimensions from an axial centerline of the frame.
50. The method of any one of embodiments 47-49 wherein the inner struts each comprise two or more recessed portions located at respective pivot joints and the outer struts are seated in the two or more recessed portions of the inner struts at respective pivot joints.
51. The method of any one of embodiments 47-50, wherein the frame further comprises pins that couple the recessed portions of the inner struts to the seated segments of the outer struts at the pivot joints.
52. The method of any one of embodiments 47-51, wherein the recessed portions have a depth that is about equal to a thickness of the segments seated in the recessed portions.
53. The method of any one of embodiments 47-52, wherein the recessed portions have a width that is greater that a width of the segments seated in the recessed portions.
54. The method of any one of embodiments 47-53, wherein at least some of the inner struts comprise recessed end portions, and end segments of the outer struts are seated in the recessed end portions forming pivot joints at apices of the frame.

## Claims

1. A prosthetic heart valve comprising:
a radially expandable and compressible annular frame (10) comprising a plurality of interconnected struts (40a; 40b; 40c; 40d; 40f; 40g; 40h; 40i; 50) and
a valvular structure mounted within the frame (10) that regulates blood flow through the prosthetic heart valve;
wherein at least some of the plurality of struts (40a; 40b; 40c; 40d; 40f; 40g; 40h; 40i; 50) comprise recessed portions (42a; 42b, 52) at respective interconnection points (J1; J2; J3) between respective two struts of the plurality of struts (40a; 40b; 40c; 40d; 40f; 40g; 40h; 40i; 50), the recessed portions comprising segments of the respective two struts, wherein the segments have a substantially continuous inner or outer surface.

2. The prosthetic heart valve of claim 1, wherein the annular frame (10) is a balloon-expandable frame.

3. The prosthetic heart valve of claim 1, wherein the annular frame (10) is a mechanically-expandable frame.

4. The prosthetic heart valve of claim 3, wherein
the plurality of struts (40a; 40b; 40c; 40d; 40f; 40g; 40h; 40i; 50) comprises inner struts (40b; 40c) and outer struts (40a) that are discrete from one another, wherein the outer struts (40a) overlap adjacent inner struts (40b; 40c) at pivot joints (J1; J2; J3) serving as interconnection points (J1; J2; J3), the recessed portions (42a; 42b, 52) provided at the pivot joints (J1; J2; J3), and radial expansion or compression of the annular frame (10) causes the inner struts (40b; 40c) to pivot relative to the outer struts (40a) at the pivot joints (J1; J2J J3); wherein
the at least some of the plurality of struts (40a; 40b; 40c; 40d; 40f; 40g; 40h; 40i; 50) comprising recessed portions (42a; 42b; 52) comprise either the outer struts (40a) or the inner struts (40b; 40c), wherein segments of the other one of the inner struts (40b; 40c) or the outer struts (40a) are seated in the recessed portions (42a; 42b; 52).

5. The prosthetic heart valve of claim 4, where the segments seated in the recessed portions (42a; 42b; 52) have a generally continuous inner surface, such that inner surfaces of the seated segments and adjacent inner surfaces the struts (40a; 40b; 40c; 40d; 40f; 40g; 40h; 40i; 50) joined to the seated segments have about equal radial dimensions from a centerline of the frame (10).

6. The prosthetic heart valve of claim 4, where the segments seated in the recessed portions (42a; 42b; 52) have a generally continuous outer surface, such that outer surfaces of the seated segments and adjacent outer surfaces the struts (40a; 40b; 40c; 40d; 40f; 40g; 40h; 40i; 50) joined to the seated segments have about equal radial dimensions from a centerline of the frame (10).

7. The prosthetic heart valve of any one of claims 3-6, wherein the annular frame (10) further comprises pins that couple the recessed portions (42a; 42b; 52) to the segments seated in the recessed portions at the pivot joints (J1; J2; J3).

8. The prosthetic heart valve of any one of claims 1-7, wherein at least some of the struts (40a; 40b; 40c; 40d; 40f; 40g; 40h; 40i; 50) comprise two or more recessed portions (42a; 42b; 52) located at respective interconnection points (J1; J2; J3) and segments of the struts (40a; 40b; 40c; 40d; 40f; 40g; 40h; 40i; 50) are seated in the two or more recessed portions (42a; 42b; 52) at the respective interconnection points (J1; J2; J3).

9. The prosthetic heart valve of any one of claims 1-8, wherein the recessed portions (42a; 42b; 52) have a depth that is about equal to a thickness of the segments seated in the recessed portions (42a; 42b; 52).

10. The prosthetic heart valve of any one of claims 1-9, wherein the recessed portions (42a; 42b; 52) have a width that is greater that a width of the segments seated in the recessed portions (42a; 42b; 52).

11. The prosthetic heart valve of any one of claims 1-10, wherein leaflets (20) of the valvular structure are attached to the struts (40a; 40b; 40c; 40d; 40f; 40g; 40h; 40i; 50) that include the recessed portions (42a; 42b; 52).

12. The prosthetic heart valve of any one of claims 1-11, wherein at least some of the struts (40a; 40b; 40c; 40d; 40f; 40g; 40h; 40i; 50) comprise recessed end portions (42a; 42b; 52) and end segments of others of the struts (40a; 40b; 40c; 40d; 40f; 40g; 40h; 40i; 50) are seated in the recessed end portions (42a; 42b; 52) forming interconnection points at apices of the frame (10).

13. The prosthetic heart valve of any one of claims 1-12, wherein leaflets (20) of the valvular structure are attached to a skirt portion at a scalloped suture line and an outer skirt (30), the leaflets and the outer skirt being attached to the frame (10) via suture loops (32a, 32b).

14. The prosthetic heart valve of claim 13, wherein the leaflets (20) contact an inner surface of the frame (10) and the outer skirt (30) contacts an outer surface of the frame (10).

15. The prosthetic heart valve of any one of claims 1-12, wherein leaflets (20) of the valvular structure are connected to the frame (10) via an inner skirt.
